# EUROPÄISCHE PATENTANMELDUNG

(11) **EP 0 783 052 A2**
(43) Veröffentlichungstag der Anmeldung: **09.07.1997**
(21) Anmeldenummer: 96119845.4
(22) Anmeldetag: 11.12.1996
(51) Int. Cl.: D06P 1/62, D06M 13/355, D06M 13/262

(54) **Flüssigformulierung von N-(2-Sulfatoethyl)piperazin-sulfat**

(30) Priorität: 20.12.1995 DE 19547649
(71) Anmelder: HOECHST AKTIENGESELLSCHAFT, 65929 Frankfurt am Main (DE)
(72) Erfinder: Schrell, Andreas, Dr., 65929 Frankfurt (DE); Meier, Michael, Dr., 65835 Liederbach (DE); Kautz, Heinz Georg, 63633 Bierstein (DE)

(57) **Zusammenfassung**

Eine 5 gew.-%ige bis gesättigte wäßrige Lösung von N-(2-Sulfatoethyl)piperazin-sulfat mit einem pH-Wert zwischen 4 und 0 kann zum Modifizieren von Fasermaterial eingesetzt werden.

## Beschreibung

Die vorliegende Beschreibung betrifft eine wäßrige Flüssigformulierung von N-(2-Sulfatoethyl)piperazin-sulfat und deren Verwendung zum Modifizieren von Fasermaterial.

N-(2-Sulfatoethyl)piperazin-sulfat ist aus DE-A-42 39 182 bekannt und kann als Mittel zur Vorbehandlung und Modifizierung von Fasermaterialien, wie synthetischem Polyamid oder Polyurethanfasermaterialien, Wolle, Seide oder Cellulosefasermaterialien, für das anschließende Färben mit anionischen Farbstoffen, insbesondere Reaktivfarbstoffen, eingesetzt werden (EP-A-0 546 476). Sei der in DE-A-42 39 182 beschriebenen Herstellungsmethode dieser Verbindung sind große Menge Ethanol notwendig, die dieses Herstellungsverfahren ökologisch und ökonomisch sehr ungünstig gestalten. Darüberhinaus kommt es dabei zur Bildung unerwünschter Nebenprodukte, die die technische Anwendung stark beeinträchtigen.

Es bestand daher ein dringendes Bedürfnis nach einer Herstellungsmethode, bei der die genannten Nachteile vermieden werden. Diese Aufgabe konnte überraschenderweise durch eine wäßrige alkoholfreie Flüssigformulierung dieser Verbindung gelöst werden.

Gegenstand der Erfindung ist eine 5 gew.-%ige bis gesättigte wäßrige Lösung von N-(2-Sulfatoethyl)piperazin-sulfat mit einem pH-Wert zwischen 4 und 0. Es ist wirtschaftlich zweckmäßig, den Gehalt an N-(2-Sulfatoethyl)piperazin-sulfat so groß wie möglich, d.h. bis zur oder annähernd der Sättigungskonzentration, einzustellen.

Bevorzugt ist daher eine wäßrige Lösung mit einem Gehalt von 25 bis 65 Gew.-%, vorzugsweise 30 bis 60 Gew.-%, besonders bevorzugt 35 bis 54 Gew.-%, insbesondere bevorzugt 40 bis 52 Gew.-%, an N-(2-Sulfatoethyl)piperazin-sulfat. Um eine Ausfällung bei tiefen Temperaturen zu vermeiden, ist es vorteilhaft, die Sättigungskonzentration bei Raumtemperatur etwas zu unterschreiten.

Damit N-(2-Sulfatoethyl)piperazin-sulfat in wäßriger Lösung auch für längere Zeit stabil bleibt, ist ein niedriger pH-Wert von Vorteil, besonders pH 0 bis 3, insbesondere pH 0,1 bis 2, ganz besonders bevorzugt pH 0,3 bis 1.

Gegenstand der Erfindung ist auch ein Verfahren zur Herstellung der erfindungsgemäßen Lösung, dadurch gekennzeichnet, daß man N-(2-Hydroxyethyl)piperazin mit einem Sulfiermittel, welches 70 bis 100 gew.-%ige Schwefelsäure oder SO₃ oder Oleum oder Chlorsulfonsäure oder eine Mischung davon ist, insbesondere 90 bis 98 gew.-%ige Schwefelsäure, umsetzt und das Reaktionsgemisch in so viel Wasser einträgt, daß die erfindungsgemäße wäßrige Lösung, gegebenenfalls unter Zugabe einer anorganischen Base, wie Alkalihydroxid oder Alkalicarbonat, entsteht.

N-(2-Hydroxyethyl)piperazin und seine Herstellung sind allgemein bekannt und beispielsweise als 95 bis 98 gew.-%-iges Handelsprodukt erhältlich.

Es ist vorteilhaft, die Umsetzung bei Temperaturen von 120 bis 220°C, vorzugsweise 140 bis 180°C, durchzuführen, so daß das entstehende Reaktionswasser entfernt wird. Es ist weiterhin vorteilhaft, die Umsetzung bei vermindertem Druck, beispielsweise bei 0,1 bis 200 mbar, durchzuführen. Das viskose Reaktionsgemisch kann nach beendeter Umsetzung direkt in Wasser eingetragen werden.

Das Sulfiermittel wird zweckmäßigerweise in einer Menge von 1,6 bis 2,2 Gewichtsteilen, vorzugsweise 1,8 bis 2,0 Gewichtsteilen, je Gewichtsteil N-(2-Hydroxyethyl)piperazin eingesetzt. Es können auch größere Mengen der genannten Sulfiermittel verwendet werden, dies gibt jedoch keine Qualitätsvorteile, und es müssen bei der späteren Anwendung lediglich größere Mengen Säure neutralisiert werden.

In einer besonders vorteilhaften Ausführungsform wird 96 gew.-%ige Schwefelsäure vorgelegt und N-(2-Hydroxyethyl)piperazin so zugegeben, daß eine Temperatur von 150°C nach Möglichkeit nicht überschritten wird. Das Reaktionsgefäß wird dann langsam evakuiert und das entsprechende Reaktionswasser bei etwa 150°C abdestilliert. Nach beendeter Reaktionszeit läßt man das noch heiße Sulfiergemisch in eine Eis/Wasser-Mischung einlaufen und stellt mit Natronlauge auf einen pH von 0,5. Man erhält so eine Lösung von Natriumsulfat und der gewünschten Substanz in Wasser, die außer geringen Anteilen an Ausgangssubstanz ausschließlich N-(2-Sulfatoethyl)piperazin-sulfat enthält. Nebenprodukte, wie sie bei der Synthese gemäß DE-A-42 39 182 entstehen, sind nicht enthalten.

Die Lösung, die man nach der Teilneutralisation der Schwefelsäure erhält, kann direkt zum Modifizieren cellulosehaltiger Fasermaterialien verwendet werden, wie es auch in der DE-A1-4402210 beschrieben ist.

In den nachfolgenden Beispielen bedeuten Prozente Gewichtsprozente.

### Beispiel

In einem 750 ml Kolben mit Rührer, Tropftrichter, Thermometer und Destillationsbrücke werden 360 g Schwefelsäure, 96%ig, vorgelegt und 195,1 g (1,5 mol) N-(2-Hydroxyethyl)piperazin, 98%ig, über ca. 20 Minuten so zugegeben, daß die Temperatur nicht über 150°C steigt. Hierbei kommt es erst zu Nebel- und dann zu Kondenswasserbildung. Anschließend wird in 30 Minuten auf 10 mbar evakuiert und 5 Stunden bei 150°C nachgerührt. In einer Aceton-Kohlendioxid-Kältefalle werden 39,8 g Wasser kondensiert. Das Reaktionsgemisch wird heiß in einen Tropftrichter überführt und in 210 g Wasser so eindosiert, daß 30°C nicht überschritten werden. Dann wird mit 143,1 g Natronlauge, 50%ig, ein pH-Wert von 0,5 eingestellt. Nach beendeter Zugabe wird 30 Minuten bei 25°C nachgerührt und vom eventuell ungelösten Anteil abfiltriert. Der Filterkuchen wird mit 30 g Wasser gewaschen. Es fallen 0,1 g Filterrückstand und 898,8 g = 564 ml N-(2-Sulfatoethyl)piperazin-sulfat-Lösung als hellbraune Flüssigkeit an. Der Gehalt nach Titration beträgt ca. 51 %, dies entspricht 457,9 g (1,49 mol) N-(2-Sulfatoethyl)piperazin-sulfat, 100%ig gerechnet, und einer Ausbeute von 99 % der Theorie.

### Anwendungsbeispiel

In einer kontinuierlichen Arbeitsweise wird Baumwoll-Interlock als Schlauch bei 100°C in ein Bad, das 1 %, bezogen auf die Flüssigkeitsmenge, einer 50 %igen Natronlauge und 1 %, bezogen auf Flüssigkeitsmenge, einer 35 %igen Wasserstoffperoxidlösung enthält, getaucht. Die Ware läßt man 10 min verweilen und zieht sie dann durch ein Quetschwerk, so daß ein Flottenauftrag von ca. 80 % verbleibt. Anschließend führt man das Material durch einen Netztrog mit Mehrfachquetschwerk, in dem sich eine Lösung befindet, die pro Liter 210 g N-(2-Sulfatoethyl)-piperazinsulfat, 50 %ig in Wasser aus dem vorstehenden Beispiel, und 210 ml einer 50 %igen Natronlauge enthält. Bei diesem Vorgang wird das Material mit einem Flottenauftrag von 110 % imprägniert. Zur weiteren Behandlung fährt der Warenstrang dann in eine J-Box und verbleibt hierin über 30 Minuten bei einer Temperatur zwischen 95 und 98°C. Anschließend wäscht man gründlich aus, befreit die Ware dabei von gelösten Schmutzpartikeln und überschüssigem Alkali und stellt auf eine Restfeuchte von 100 % ein. Das noch feuchte Material kann danach direkt nach einer Ausziehmethode in einer Düsenfärbemaschine gefärbt werden. Dazu werden 1000 Teile des Materials mit 10000 Teilen Wasser versetzt.

Anschließend dosiert man 20 Teile eines Farbstoffs der allgemeinen Formel 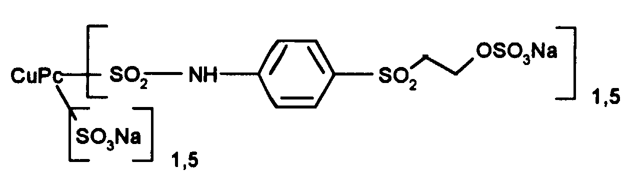 bekannt aus der DE-A1-1179317, Beispiel 2, über eine Zeit von 10 Minuten zu. Man heizt auf 80°C auf und beläßt das Material bei dieser Temperatur über 45 Minuten. Anschließend kühlt man auf 60°C, läßt die Restflotte ab und behandelt das Material nach gängigen Methoden nach. Nach dem Trocknen erhält man eine farbstarke Türkisfärbung mit guten Gebrauchsechtheiten.

## Patentansprüche

1. Eine 5 gew.-%ige bis gesättigte wäßrige Lösung von N-(2-Sulfatoethyl)piperazin-sulfat mit einem pH-Wert zwischen 4 und 0.

2. Wäßrige Lösung nach Anspruch 1, dadurch gekennzeichnet, daß der Gehalt an N-(2-Sulfatoethyl)piperazin-sulfat 25 bis 65 Gew.-%, insbesondere 40 bis 52 Gew.-%, beträgt.

3. Wäßrige Lösung nach Anspruch 1 oder 2, dadurch gekennzeichnet, daß der pH-Wert der Lösung zwischen 3 und 0, insbesondere zwischen 1 und 0,3 ist.

4. Verfahren zur Herstellung einer wäßrigen Lösung nach mindestens einem der Ansprüche 1 bis 3, dadurch gekennzeichnet, daß man N-(2-Hydroxyethyl)piperazin mit einem Sulfiermittel, das 70 bis 100 gew.-%ige Schwefelsäure oder Oleum oder Schwefeltrioxid oder Chlorsulfonsäure oder eine Mischung davon ist, umsetzt und das Reaktionsgemisch in so viel Wasser, gegebenenfalls unter Zugabe einer anorganischen Base, einträgt, daß die besagte wäßrige Lösung entsteht.

5. Verfahren nach Anspruch 4, dadurch gekennzeichnet, daß man 90 bis 98 gew-%ige Schwefelsäure einsetzt.

6. Verfahren nach Anspruch 4 oder 5, dadurch gekennzeichnet, daß man das Sulfiermittel in einer Menge von 1,6 bis 2,2 Gewichtsteilen, vorzugsweise 1,8 bis 2,0 Gwichtsteilen, je Gewichtsteil N-(2-Hydroxyethyl)piperazin einsetzt.

7. Verfahren nach mindestens einem der Ansprüche 4 bis 6, dadurch gekennzeichnet, daß die Umsetzung bei einer Temperatur zwischen 120 und 220°C, bevorzugt zwischen 140 und 180°C, durchgeführt wird.

8. Verfahren nach mindestens einem der Ansprüche 4 bis 7, dadurch gekennzeichnet, daß die Umsetzung bei vermindertem Druck durchgeführt wird.

9. Verwendung einer wäßrigen Lösung nach einem oder mehreren der Ansprüche 1 bis 3 zum Modifizieren von cellulosehaltigen Fasermaterialien.
